# EUROPEAN PATENT SPECIFICATION

(11) **EP 4 259 271 B1**
(45) Date of publication and mention of the grant of the patent: **19.03.2025**
(21) Application number: 21824487.9
(22) Date of filing: 24.11.2021
(51) Int. Cl.: A61N 1/36, A61N 1/375, H01R 13/52, H01G 9/008, A61N 1/378, A61N 1/39, H01G 9/048, H01M 10/04, H01M 10/42

(54) **ELECTRICAL COMPONENT FOR AN IMPLANTABLE MEDICAL DEVICE**
ELEKTRISCHE KOMPONENTE FÜR EINE IMPLANTIERBARE MEDIZINISCHE VORRICHTUNG
COMPOSANT ÉLECTRIQUE POUR UN DISPOSITIF MÉDICAL IMPLANTABLE

(30) Priority: 11.12.2020 EP 20213359
(43) Date of publication of application: 18.10.2023
(73) Proprietor: BIOTRONIK SE & Co. KG, 12359 Berlin (DE)
(72) Inventor: WEISS, Ingo, 12435 Berlin (DE); DÖRR, Thomas, 12437 Berlin (DE)
(74) Representative: Biotronik Corporate Services SE
(86) International application number: PCT/EP2021/082861
(87) International publication number: WO 2022/122390

(56) References cited:
- EP-A1- 3 284 515
- WO-A1-2010/148379
- US-A- 5 679 026
- US-A- 5 930 109
- US-A1- 2004 068 302
- US-A1- 2008 170 353

## Description

The present invention relates to an electrical component for an implantable medical device according to the preamble of claim 1.

An electrical component of this kind comprises an outer shell forming a first side face and a second side face, a functional element enclosed in the outer shell, and an arrangement of connection devices arranged on the outer shell for establishing an electrical connection of the functional element to a component of the implantable medical device outside of the outer shell.

An implantable medical device may for example have the shape of a leadless stimulation device, such as a leadless pacemaker device. In this case a housing of the leadless pacemaker device encapsulates components of the leadless pacemaker device such as a processor, a data memory, a battery and other processing equipment to allow for operation of the leadless pacemaker device in an autarkic manner. The leadless pacemaker device may be implanted directly into the heart and may operate within the heart, for example within the right ventricle of the heart, without requiring any leads for placing an electrode at a location of interest within the heart.

The implantable medical device may also be a stimulation device which comprises a generator to be implanted for example subcutaneously at a location remote from the heart. In this case e.g. a lead extends from the generator into the heart to allow for a stimulation or a sensing of signals at a location of interest within the heart, for example within the right ventricle.

Generally, the implantable medical device may be any device which, in an implanted state, provides for a diagnostic or therapeutic function within the patient, such as a stimulation function, a sensing function, a monitoring function, a recording function or the like, wherein the implantable medical device is not limited to cardiac applications, but for example may also be used for neuro-applications.

An implantable medical device of this kind, for example a cardiac stimulation device such as a subcutaneous CRT device or a leadless pacemaker device, generally shall be implanted into the patient over a prolonged period of time, such that the implantable medical device remains operative within the patient over its lifespan. For this, the implantable medical device comprises electrical components, such as an energy storage device in the shape of a battery or a capacitor, which shall power the implantable medical device for its operation.

Implantable medical devices of this kind shall be small in built such that they are easily implantable, for example subcutaneously or immediately within the patient's heart or in another vessel of interest. This requires electrical components to be compact and space-efficient. In addition, however, there is a desire to manufacture such electrical components in a cost-efficient manner while ensuring reliable operation of the implantable medical device over its lifespan.

An energy storage device of an implantable medical device for example is formed by an assembly of electrical components, for example battery or capacitor components, which are assembled such that they together form the energy storage device. As the assembly of electrical components shall be tightly fit into a housing of the implantable medical device, for example the housing of a subcutaneous generator or the housing of a leadless pacemaker device, the different electrical components generally need to be manufactured differently, such that a variety of different electrical components exists which need to be designed and manufactured separately. This adds to the costs and also to the complexity of the manufacturing.

In document WO 2010/148379 A1 a hub is described including a first lead receptacle having a plurality of contacts for electrically coupling a lead to an implantable electrical device. The hub further contains a second lead receptacle having a plurality of contacts for electrically coupling a lead to the implantable electrical device. At least one of the plurality of contacts of the first receptacle is a contact of the second receptacle. Such a configuration may allow for the overall size of the hub to be reduced relative to a hub where each discrete contact of the hub corresponds to a discrete contact or electrical channel ofthe implantable electrical device.

In document EP 3 284 515 A1 a composite is described comprising a ceramic body, comprising a) a ceramic; b) a first surface; c) a hole, comprising a front face, an end face and a lateral surface, wherein the front face is an opening in the first surface; d) a second surface; and e) a conductor a1, wherein the conductor a1 i) electrically connects the second surface to the lateral surface,and ii) comprises a cermet. Furthermore, a process for producing a ceramic body is described comprising a plurality of cermet conductors; to another process for producing a ceramic body comprising a plurality of cermet conductors; to another process for producing a ceramic body comprising a plurality of cermet conductors; to yet another process for producing a ceramic body comprising a plurality of cermet conductors; to a composite obtainable by said processes; to a device comprising one of the above composites; to a use of one of the above composites to electrically connect an electrode to an implantable electrical medical device; to a process of implanting the above device into an eukaryotic organism; and to a use of the above device in a therapy.

It is an object of the instant invention to provide an electrical component for an implantable medical device and an implantable medical device which allow for an easy manufacturing and assembling of the implantable medical device.

This object is achieved by means of an electrical component comprising the features of claim 1.

Accordingly, a first of the connection devices is arranged on the first side face (of the outer shell of the electrical component) and a second of the connection devices is arranged on the second side face, wherein the electrical component is configurable to assume different configurations states, wherein in a first configuration state the first of the connection devices is configured to establish said electrical connection and the second of the connection devices is deactivated, and in a second configuration state the first of the connection devices is deactivated and the second of the connection devices is configured to establish said electrical connection.

The electrical component may be configured to assume different configurations states. Herein, the configuration states may at least include a first configuration state and a second configuration state, wherein the configuration states differ in the configuration of the connection devices for establishing an electrical connection to the functional element enclosed in the outer shell.

Hence, the electrical component is enabled such that it may brought in any of the configuration states. Dependent on the particular configuration state for which the electrical component is configured, the first of the connection devices is used to establish the electrical connection to the functional element (while the second of the connection devices is deactivated), or the second of the connection devices is used to establish the electrical connection to the functional element (while the first of the connection devices is deactivated).

The outer shell forms a housing for the electrical component. Herein, two side faces are formed on the outer shell, wherein on each side face (at least) one connection device is arranged. Any of the connection devices herein may be used to establish a connection to the functional element enclosed in the outer shell, such that e.g. depending on the placement of the electrical component within the implantable medical device one or another connection device of the first or the second side face may be used to establish the electrical connection.

By providing different connection devices on different side faces it becomes possible to form an assembly of electrical components, for example for providing for an energy storage device within an implantable medical device, which is assembled of electrical components having outer shells of identical shape and structure. Hence, for example for providing for an energy storage device, electrical components of uniform structure and shape may be provided, such that the manufacturing and the assembly of the implantable medical device may be eased. In particular, the variety of parts for constructing the implantable medical device is reduced, hence also reducing requirements for the supply chain and the complexity of the manufacturing.

Depending on the configuration a connection device on one side face is used to establish the electrical connection between the external component of the implantable medical device and the functional element included in the outer shell, whereas the connection device on the other side face is deactivated. This means that the other connection device is not used for establishing an electric connection, but is electrically deactivated and, for example, is closed such that no fluid may enter into the outer shell via the connection device.

In one embodiment, the connection devices are formed by through-openings. For establishing the electrical connection to the functional element included in the outer shell, hence, a connection line may be passed through one of the connection devices, such that an electrical connection in between the connection line and the functional element is established within the outer shell.

In another embodiment, the connection devices may be formed by plug connectors which may be connected to connection lines by forming plug connections, i.e. by plugging the connection lines into the plug connectors.

In one embodiment, the electrical component comprises a closure element for deactivating the first or the second of the connection devices, depending on the configuration state. The closure element serves to close the associated connection device, in particular when the connection device is shaped as a through-opening which provides for an opening to the inside of the outer shell. By fitting the closure element to the desired connection device, the connection device, for example in the shape of a through-opening or a plug connector, is closed, such that no fluid may enter into the inside of the outer shell via the connection device.

For establishing the electrical connection in between the external component of the implantable medical device and the functional element enclosed within the outer shell, a connection line may in one embodiment be passed through one of the connection devices, depending on the configuration state, wherein the other connection device is closed for example by a suitable closure element. Herein, in order to pass the connection line into the inside of the outer shell and to for example seal the connection device (through which the connection line is passed) such that no fluid may enter into the inside of the outer shell, a guide element may be provided, the guide element serving to guide the connection line through the associated connection device. The guide element may for example be fitted into the connection device, for example in the shape of a through-opening. The guide element may comprise a bore through which the connection line is passed, wherein a transition in between the connection line and the outer shell is sealed by means of the guide element.

Such guide element may for example be formed from a glass element, which may be fitted into one of the connection devices, depending on the configuration state of the electrical component.

The functional element enclosed within the outer shell is for example connected to the connection line within the outer shell, for example at a connection location which is arranged symmetrically with respect to the first side face and the second side face. In particular, the connection location may be placed symmetrically with respect to the connection devices arranged on the side faces of the outer shell. This allows to pass the connection line through any of the connection devices towards the connection location within the outer shell, without the connection to the functional element being influenced by the use of the first connection device or the second connection device for establishing the connection.

In one embodiment, the first side face and the second side face are formed and arranged such that the first side face and the second side face are symmetric with respect to each other relative to a plane of symmetry. The first side face and the second side face hence have a symmetric shape, in that the first side face equals the second side face when mirrored with respect to the plane of symmetry.

Herein, in one embodiment, the first of the connection devices and the second of the connection devices are symmetric with respect to each other relative to the plane of symmetry.

By forming the side faces and, beneficially, also the connection devices on the side faces, in a symmetrical fashion, it becomes possible to use the electrical component in different placements and orientations. For example, in one orientation the first side face may face in a particular direction, and in another orientation of the electrical component the second side face may face in the same direction. This, hence, allows to combine electrical components of equal shape in a suitable assembly of electrical components, with side faces of the electrical components being aligned to extend in a common plane, wherein depending on the placement and orientation of the electrical components one side face or the other side face of each component comes to rest in the common plane. By configuring the different electrical components to establish the electric connection using the first connection device or the second connection device, an electrical connection to the different electrical components in the same, common plane may be established.

In one embodiment, the outer shell forms a planar abutment face for placing the electrical component on another, second electrical component, wherein the plane of symmetry is perpendicular to the abutment face. One electrical component, via its planar abutment face, hence can be placed on and fastened to another electrical component. In an assembled state, the electrical components with their abutment faces come to rest on each other, such that the electrical components may be connected to each other, for example by gluing or welding.

The side faces may for example extend perpendicularly to the planar abutment face and herein are symmetric to the plane of symmetry, which is also perpendicular to the abutment face.

In one embodiment, the first side face and the second side face each have a planar shape and hence extend straight, for example perpendicularly to the abutment face.

For example, the first side face and the second side face may be arranged at an angle with respect to each other, for example a right angle. The plane of symmetry, in this case, is arranged along the bisector plane, e.g. at 45°, with respect to the side faces.

An implantable medical device, for example a stimulation device such as a cardiac stimulation device, for example a CRT device which is to be implanted subcutaneously in a patient or a leadless pacemaker device, for example comprises an assembly of electrical components. The electrical components herein may form a functional group within the implantable medical device, for example to provide an energy storage device within the implantable medical device. The electrical components herein, for example, may be battery or capacitor elements, which may be assembled to form the energy storage device.

In one embodiment, in an assembly of a multiplicity of electrical components at least some of the electrical components comprise outer shells of identical shape, such that electrical components of a uniform structure and shape may be used to form the assembly. Within the assembly the electrical components are arranged to abut one another, wherein the electrical components for example may be connected to each other by gluing or welding to form a cohesive assembly for integration into the implantable medical device.

In one embodiment, four electrical components are combined to form the assembly. The electrical components may be equal in shape and structure. In the assembly, however, the electrical components differ in their orientation in in their configuration state, such that depending on the orientation of a particular electrical component the first or the second configuration state is applied for establishing an electrical connection to the respective electrical component.

In one embodiment, side faces of the electrical components of the assembly group are arranged to extend along a common plane. Herein, a first of the electrical components may lie in the common plane with a first side face, wherein a second of the electrical components lies in the common plane with a second side face. Depending on whether the first side face or the second side face of the particular electrical component lies in the common plane, the first configuration state or the second configuration state is used, such that in any case the electrical connection to the particular electrical component may be established in the common plane, hence easing the electrical integration within the implantable medical device.

The idea of the invention shall subsequently be described in more detail with reference to the embodiments shown in the figures. Herein:
- Fig. 1: shows a schematic view of an implantable medical device in the shape of a cardiac stimulation device;
- Fig. 2: shows a schematic drawing of an implantable medical device comprising electrical components;
- Fig. 3: shows an assembly of electrical components, for example for forming an energy storage device of the implantable medical device;
- Fig. 4: shows a schematic drawing of a single electrical component;
- Fig. 5: shows a functional view of the electrical component, in a first example;
- Fig. 6: shows a functional view of the electrical component, in another example;
- Fig. 7: shows a functional view of the electrical component, in a first configuration state;
- Fig. 8: shows a functional view of the electrical component, in a second configuration state;
- Fig. 9: shows a schematic, perspective view of an electrical component, indicating a plane of symmetry between side faces of an outer shell of the electrical component; and
- Fig. 10: shows a view of two electrical components.

Subsequently, embodiments of the invention shall be described in detail with reference to the drawings. In the drawings, like reference numerals designate like structural elements.

It is to be noted that the embodiments are not limiting for the invention, but merely represent illustrative examples.

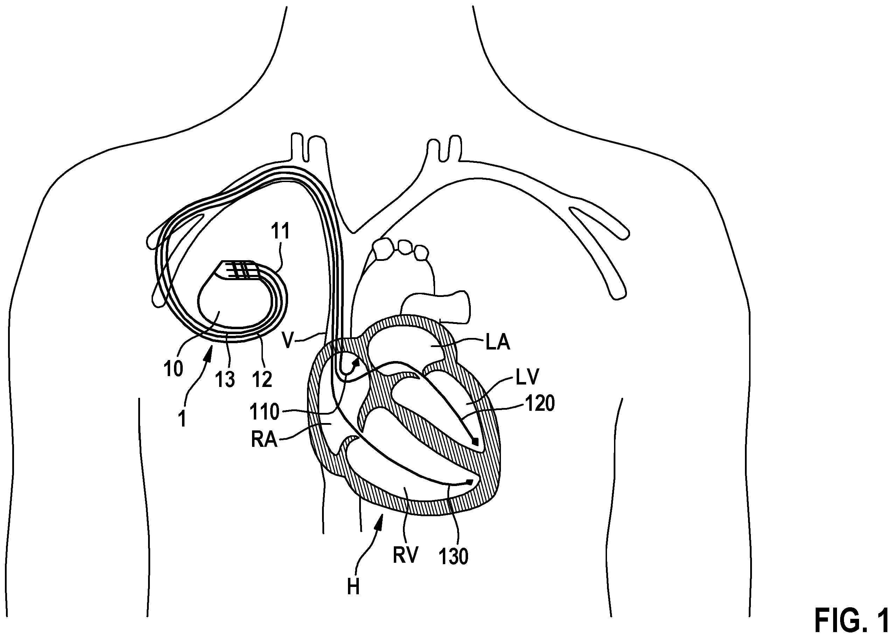

Fig. 1 shows, in a schematic drawing, the human heart comprising the right atrium RA, the right ventricle RV, the left atrium LA and the left ventricle LV. An implantable medical device 1 in the shape of a stimulation device, such as a CRT device, is implanted in a patient, the implantable medical device 1 comprising a generator 10 connected to leads 11-13 extending from the generator 10 through the superior vena V into the patient's heart. By means of the leads 11-13, electrical signals for providing a stimulation action in the heart shall be injected into intra-cardiac tissue potentially at different locations within the heart, and sense signals may be received. In addition, e.g. a defibrillation therapy may be performed by an electrode arrangement arranged on one or both of the leads 11-13.

An implantable medical device 1 as concerned herein may generally be a cardiac stimulation device such as a cardiac pacemaker device. A stimulation device of this kind may comprise a generator 10, as shown in Fig. 1, which may be subcutaneously implanted into a patient at a location remote from the heart, one or multiple leads 11-13 extending from the generator 10 into the heart for emitting stimulation signals in the heart or for obtaining sense signals at one or multiple locations from the heart.

In another embodiment, the implantable medical device 1 may be a leadless pacemaker device, which does not comprise leads, but has the shape of a capsule and may be directly implanted into the heart, for example into the right ventricle RV of the heart.

In yet another embodiment, the implantable medical device 1 may be a monitoring device, a sensing device or any other implantable medical device which shall be implanted in a patient to perform a diagnostic or therapeutic function within the patient.

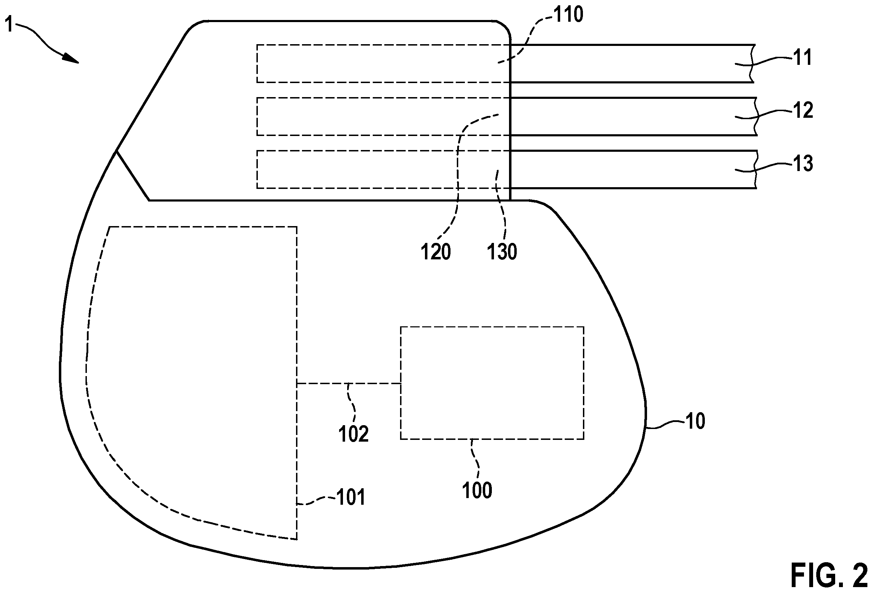

An implantable medical device 1 as concerned herein generally may remain in a patient over a prolonged period of time, wherein the implantable medical device 1 shall remain operative within the patient over its lifespan. For this, the implantable medical device 1, as schematically illustrated in Fig. 2, comprises an energy storage device 101, in particular in the shape of a battery or a capacitor arrangement, which shall supply electrical energy to functional components of the implantable medical device 1, such as a processor device 100, a communication device for communicating with external devices, sensing devices or any other equipment of the implantable medical device 1.

Implantable medical devices 1 of the kind concerned herein generally shall be small to ease their implantation and to reduce an impact on the patient. Hence, components integrated in the implantable medical device 1 necessarily need to be compact in structure and built, wherein e.g. an energy storage device 101 must have a sufficient storage capacity for storing electrical energy to ensure operation of the implantable medical device 1 over its desired lifespan.

This requires electrical components to be fitted into the housing of the implantable medical device 1 in a tight, space-efficient manner.

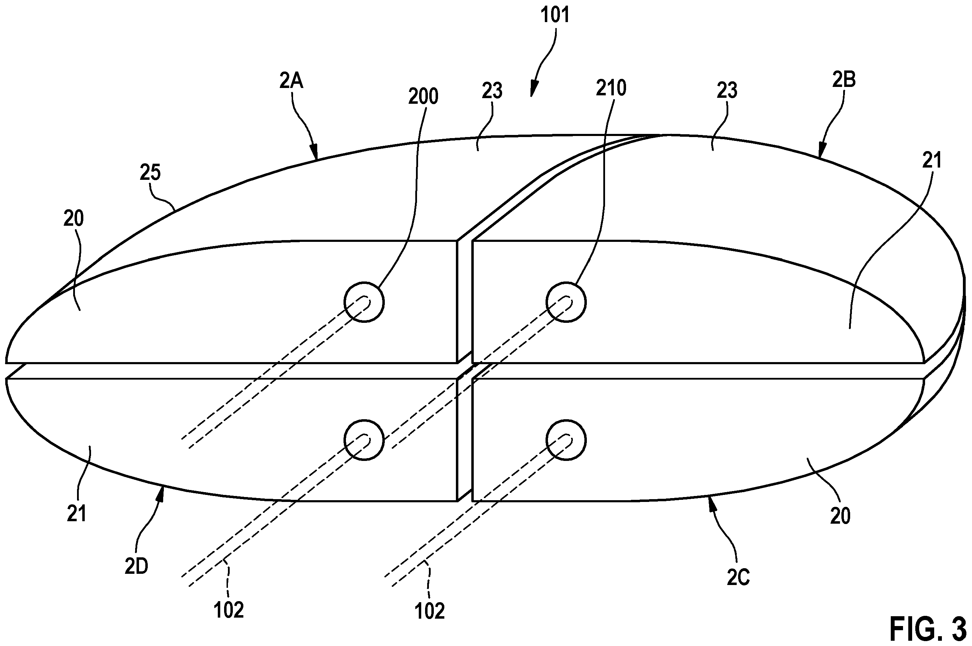

Referring now to Fig. 3, an electrical assembly, such as the energy storage device 101, may be assembled from separate electrical components 2A, 2B, 2C, 2D which are combined with each other and form an assembly which may tightly be fitted into the housing of the implantable medical device 1. For example, outer faces 23 of the electrical components 2A, 2B, 2C, 2D may together form an outer surface of the assembly, which may conform to the shape of an inner cavity of the housing of the implantable medical device 1, such that the assembly may tightly be received in the cavity.

In the example of Fig. 3, the electrical components 2A, 2B, 2C, 2D may be formed with outer shells 25 having an identical outer shape, as this shall be explained further below. Side faces 20, 21 of the electrical components 2A, 2B, 2C, 2D herein come to lie in a common plane and may face in the same direction, which allows to establish an electrical connection using connection lines 102 to the different electrical components 2A, 2B, 2C, 2D within the common plane.

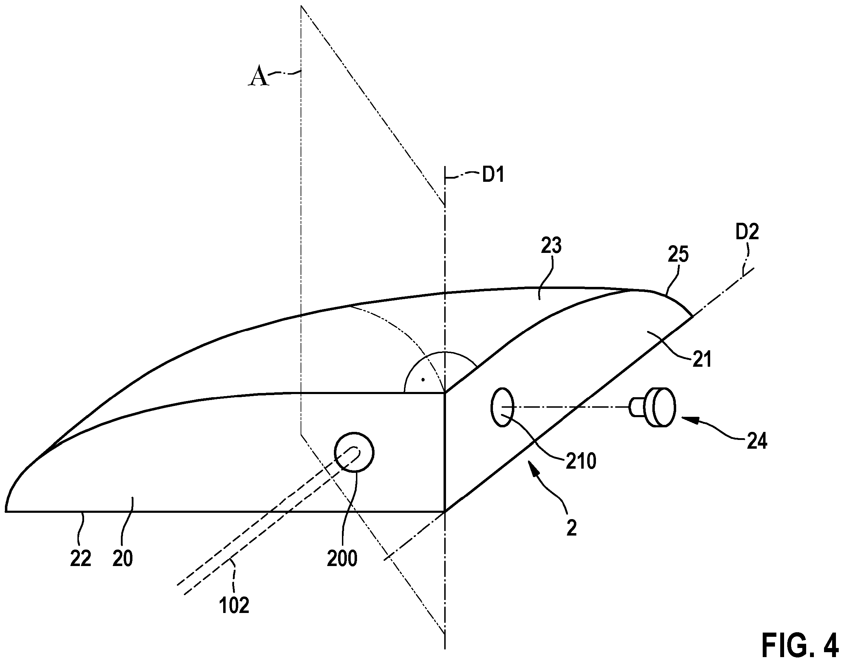

Referring now to Fig. 4, each electrical component 2A, 2B, 2C, 2D - commonly denoted in Fig. 4 with reference numeral 2 - comprises an outer shell 25, which is confined by an outer, rounded face 23, side faces 20, 21 and an abutment face 22.

The side faces 20, 21 are arranged at a right angle with respect to one another and have a straight, planar extension. The side faces 20, 21 herein are symmetrical with respect to a plane of symmetry A extending along a bisector plane in between the side faces 20, 21.

The abutment face 22 is formed by a planar, straight face and extends along a plane perpendicular to the side faces 20, 21 and the plane of symmetry A. By means of the abutment face 22 the electrical component 2 may be placed on another electrical component, as it is shown for example in Fig. 3 for the electrical components 2A, 2D. Via the abutment face 22 a connection to another electrical component may be established, for example by gluing or by welding.

The outer face 23 points outwards when the electrical component 2 is combined with other electrical components to form a combined assembly, as this is shown in Fig. 3. The outer face 23 may have a rounded shape, which however is not limiting for the invention. Generally, the outer face 23 has a shape which for example conforms to the shape of an inner cavity of a housing of the implantable medical device 1, such that an assembly formed by a multiplicity of electrical components 2 may be tightly fit into the housing of the implantable medical device 1.

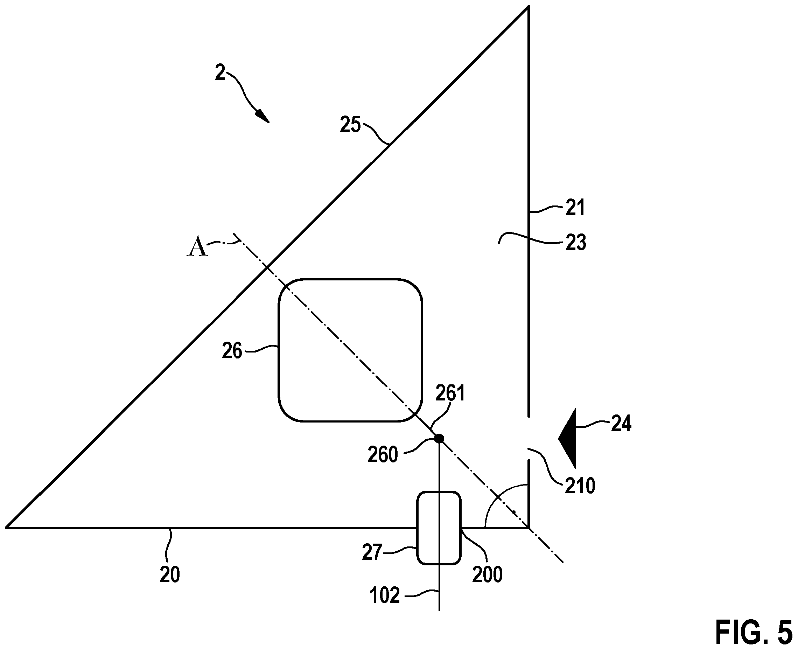

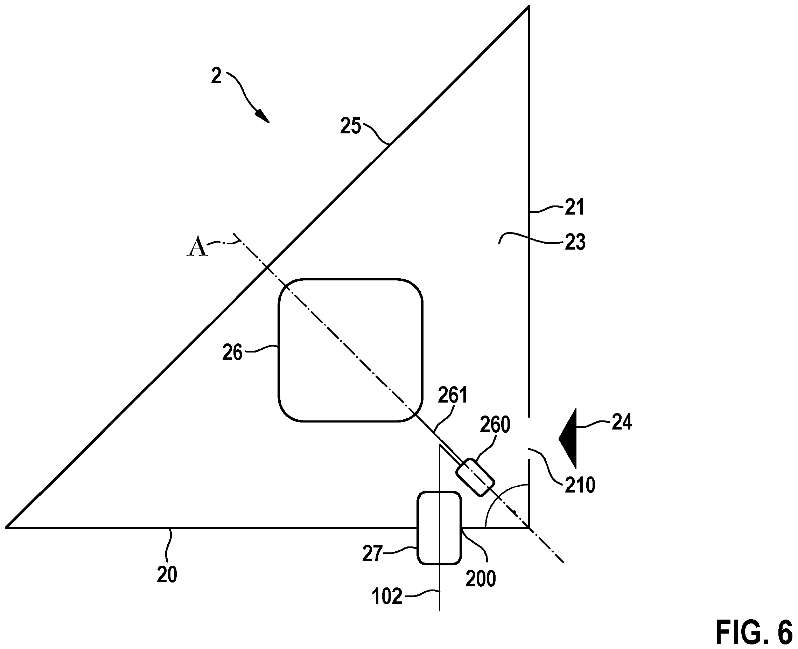

As this is visible from Fig. 4, each side face 20, 21 comprises a connection device 200, 210 in the shape of a through-opening through which a connection line 102 may be passed to establish an electrical connection to a functional element 26 enclosed within the outer shell 25, as it is shown schematically in Fig. 5 and 6.

Namely, the connection line 102 is passed through one of the connection devices 200, 210 of the side faces 20, 21 to establish an electric connection at a connection location 260 to a connector 261 of the functional element 26 within the outer shell 25. The functional element 26 may for example have the shape of an energy storage unit, such as a battery unit or a capacitor unit. At the connection location 260 the connection line 102 is electrically contacted with the connector 261, wherein the connection location 260 beneficially is arranged on the plane of symmetry A and hence is arranged symmetrically with respect to the side faces 20, 21 and the connection devices 200, 210 formed on the side faces 20, 21.

In the example of Fig. 5, the connection in between the connection line 102 and the connector 261 at the connection location 260 is formed by a point connection, for example by means of soldering.

In the example of Fig. 6, the connection in between the connection line 102 and the connector 261 at the connection location 260 is formed by a line connection, for example by welding.

The connection in between the connection line 102 and the connector 261 may for example be formed by soldering, welding or by a plug connection.

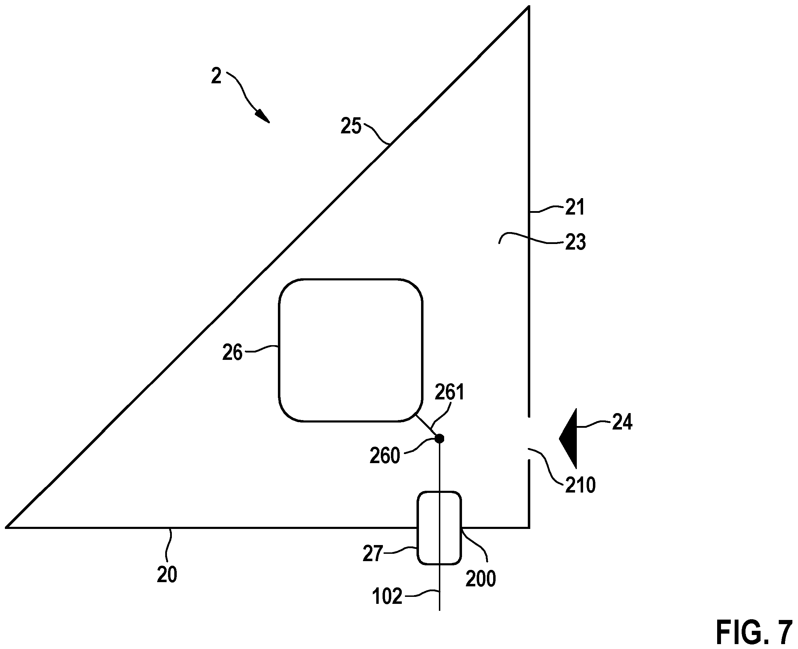

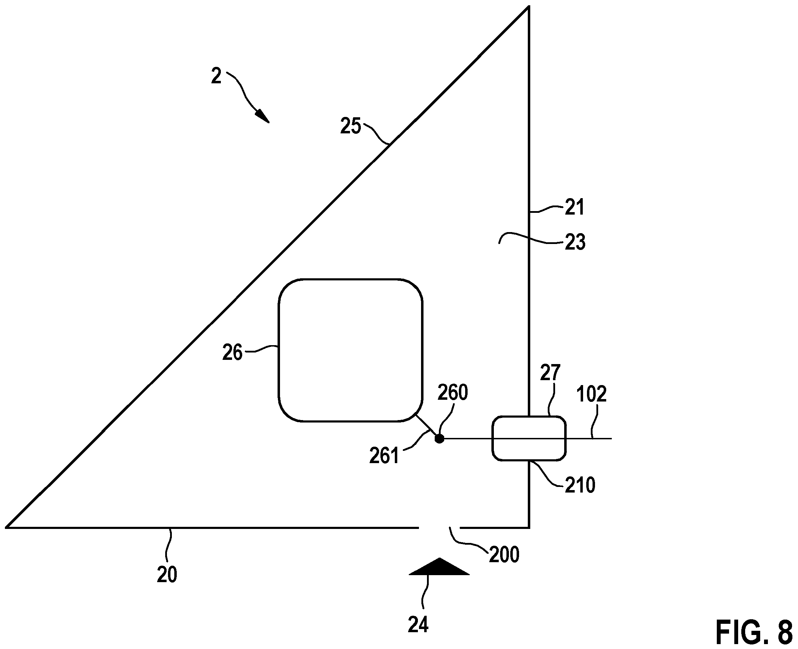

As illustrated in Figs. 7 and 8, a guide element 27 is provided to pass the connection line 102 through the respective connection device 200, 210. The guide element 27 may for example be formed by a glass element and serves to guide the connection line 102, such that a transition in between the connection line 102 and the respective side face 20, 21 is sealed in a fluid-tight, hermetic manner.

Whereas one of the connection devices 200, 210 is used to pass a connection line 102 into the outer shell 25 to establish an electrical connection to the functional element 26 enclosed within the outer shell 25, the other connection device 210, 200 shall be deactivated. For this, as schematically illustrated in Fig. 4 and likewise in Figs. 5 and 6, a closure element 24 is provided, which may be placed on any of the connection devices 200, 210 to close off the connection device 200, 210 in the shape of the through-opening, such that the connection device 200, 210 is sealed and no fluid may enter into the inside of the outer shell 25 through the respective connection device 200, 210.

By using the guide element 27 and the closure element 24, both connection devices 200, 210 in the shape of through-openings are hermetically closed in an assembled, configured state, such that no fluid may enter into the outer shell 25.

Because the side faces 20, 21 are arranged symmetrically with respect to each other and comprise symmetric connection devices 200, 210, the electrical component 2 may be configured to assume different configuration states. This is illustrated in Figs. 7 and 8.

Namely, in a first configuration state, shown in Fig. 7, the connection line 102 is passed through the connection device 200 in the shape of the through-opening on the side face 20 and is connected to the connector 261 of the functional element 26 within the outer shell 25.

In a second configuration state, shown in Fig. 8, the connection line 102 is passed through the connection device 210 in the shape of the through-opening on the side face 21 and is connected to the connector 261 of the functional element 26 within the outer shell 25.

Depending on the configuration state, hence, different connection devices 200, 210 are used to establish the electrical connection to the functional element 26 enclosed within the outer shell 25. Which configuration state is used, herein, depends on the placement and orientation of the electrical component 2 within an assembly of multiple electrical components, as this becomes clear from Fig. 3. Namely, if the electrical connection to the electrical components of the assembly shall be established in a common plane, as illustrated in Fig. 3, the electrical connection is established for each electrical component 2A-2D via that connection device 200, 210 of that side face 20, 21 which lies in the common plane.

Because the side faces 20, 21 and the connection devices 200, 210 are arranged symmetrically with respect to each other, the electrical component 2, as illustrated in Fig. 4, may be turned about rotational axes D1, D2 to transform e.g. the electrical component 2A of Fig. 3 into the electrical components 2B, 2C, 2D. Because of the symmetrical structure of the electrical components 2, hence, identical elements can be used to form the components 2A-2D of Fig. 3.

The configuration state of the electrical component 2 is hence chosen dependent on the orientation and placement of the electrical component 2 in an assembly group for integration into the implantable medical device 1. Dependent on the placement and orientation, the connection line 102 is passed through one of the connection devices 200, 210, whereas the other connection device 210, 200 is closed and hence deactivated by means of a suitable closure element 24, as it is indicated in Figs. 7 and 8.

In an initial, pre-assembly state the configuration state of the electrical component 2 is not fixed, such that the electrical component 2 may be brought into any configuration state by passing the connection line 102 through one of the connection devices 200, 210 and by closing the other connection device 210, 200. This may take place while assembling the assembly group as illustrated in Fig. 3.

At the time of passing the connection line 102 through one of the connection devices 200, 210, herein, the other connection device 210, 200 in the shape of the through-opening may still be open, such that via the opening the inside of the outer shell 25 may be accessed to e.g. form the connection in between the connection line 102 and the connector 261 at the connection location 260 within the outer shell 25. Once the connection is established the closure element 24 may be placed on the opening and the connection device 210, 200 may hence be closed and deactivated.

Prior to closing the respective connection device 210, 200 by means of the closure element 24, additional components may be introduced into the outer shell 25, or a fluid, such as a gas fluid or a liquid fluid, may be filled into the outer shell 25.

The closing of the respective connection device 210, 200 by means of the closure element 24 may be established by placing the closure element 24 in a form-fitting manner on the connection device 210, 200. Alternatively or in addition, a welding, soldering, gluing or screwing connection may be formed to fix the closure element 24 on the outer shell 25.

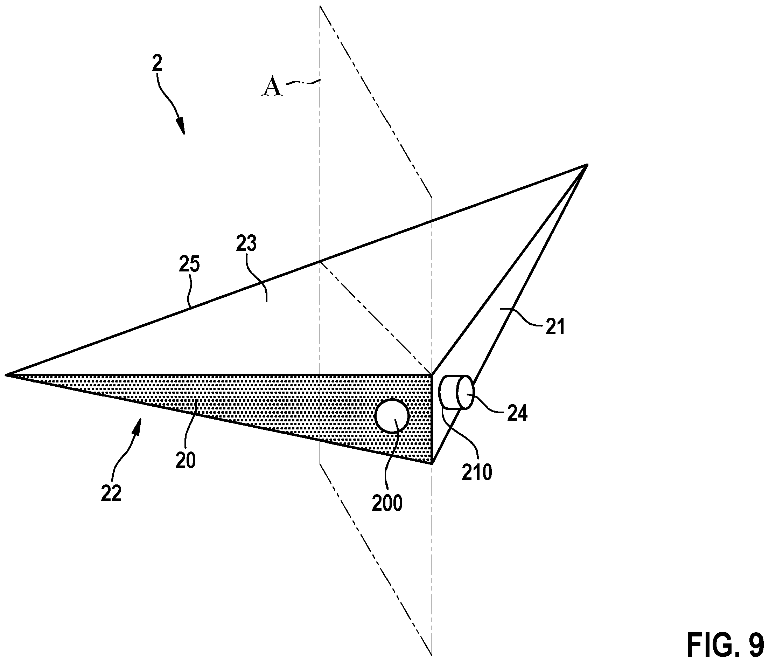

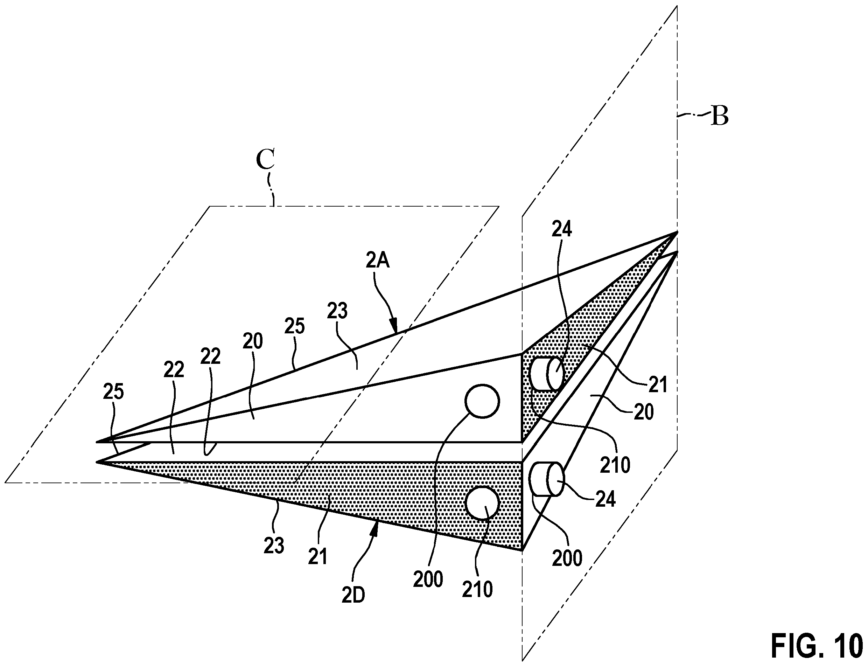

Fig. 9 - in a schematic, perspective view - illustrates the plane of symmetry A of an embodiment of an electrical component 2. As visible from Fig. 10, when placing electrical components 2A, 2B on top of each other such that the electrical components 2A, 2D abut one another with their abutment faces 22, different side faces 20, 21 lie in different planes. For example, the side face 21 of the upper electrical component 2A and the side face 20 of the lower electrical component 2D lie in a common plane B, whereas the other side faces 20, 21 lie in another common plane. Because the connection devices 210, 200 of the side faces 21, 20 lying in the plane B each are closed by a closure element 24 and hence are deactivated, the electrical components 2A, 2D are mirrored with respect to a mirror plane C, in which the abutment faces 22 of the electrical components 2A, 2D extend.

The idea of the invention is not limited to the embodiments described above.

An electrical component as described herein is used to form an energy storage device in an implantable medical device. For this, the electrical component may provide a battery component or a capacitor component for storing electrical energy.

In another embodiment not falling under the scope of the invention, the electrical component may provide another functional component, such as a processing component or a sensing component for the implantable medical device.

The electrical component, with its outer shell, may generally have any - preferably however symmetric - shape. Beneficially, the electrical component has a generally flat shape, with an abutment face having a comparatively large dimensional extension in comparison to a height by means of which side faces extend perpendicularly from the abutment face.

### List of Reference Numerals

- 1: Implantable medical device
- 10: Generator device
- 100: Processor device
- 101: Energy storage device
- 102: Connection line
- 11-13: Lead
- 110, 120, 130: Connector
- 2, 2A-D: Electrical component
- 20, 21: Side face
- 200, 210: Electrical connection device (through-opening)
- 22: Abutment face
- 23: Outer face
- 24: Closure element
- 25: Outer shell (housing)
- 26: Functional element
- 260: Connection location
- 261: Connector
- 27: Guide element
- A: Plane of symmetry
- B, C: Plane
- D1, D2: Virtual axis of rotation
- H: Heart
- LA: Left atrium
- LV: Left ventricle
- RA: Right atrium
- RV: Right ventricle
- V: Superior vena

## Claims

1. An electrical component (2, 2A-2D) for an implantable medical device (1), comprising:
an outer shell (25) forming a first side face (20) and a second side face (21),
a functional element (26) enclosed in the outer shell (25), and
an arrangement of connection devices (200, 210) arranged on the outer shell (25) for establishing an electrical connection of the functional element (26) to a component of the implantable medical device (1) outside of the outer shell (25),
wherein a first of the connection devices (200, 210) is arranged on the first side face (20) and a second of the connection devices (200, 210) is arranged on the second side face (21), wherein the electrical component (2, 2A-2D) is configurable to assume different configuration states, wherein in a first configuration state the first of the connection devices (200, 210) is configured to establish said electrical connection and the second of the connection devices (200, 210) is deactivated, and in a second configuration state the first of the connection devices (200, 210) is deactivated and the second of the connection devices (200, 210) is configured to establish said electrical connection, and
**characterised in that**
the electrical component (2, 2A-2D) is used to form an energy storage device (101) for the implantable medical device (1).

2. The electrical component (2, 2A-2D) according to claim 1, **characterized in that** the connection devices (200, 210) are formed by through-openings.

3. The electrical component (2, 2A-2D) according to claim 1 or 2, **characterized by** a closure element (24) for deactivating, depending on the configuration state, the first or the second of the connection devices (200, 210).

4. The electrical component (2, 2A-2D) according to one of claims 1 to 3, **characterized in that**, for establishing said electrical connection, a connection line (102) is passed through the first or the second of the connection devices (200, 210), depending on the configuration state.

5. The electrical component (2, 2A-2D) according to claim 4, **characterized by** a guide element (27) for guiding the connection line (102) through the first or the second of the connection devices (200, 210), depending on the configuration state.

6. The electrical component (2, 2A-2D) according to claim 4 or 5, **characterized in that** the functional element (26), at a connection location (260) inside the outer shell (25), is connected to the connection line (102), wherein the connection location (260) is arranged symmetrically with respect to the first side face (20) and the second side face (21).

7. The electrical component (2, 2A-2D) according to one of the preceding claims, **characterized in that** the first side face (20) and the second side face (21) are formed and arranged such that the first side face (20) and the second side face (21) are symmetric with respect to each other relative to a plane of symmetry (A).

8. The electrical component (2, 2A-2D) according to claim 7, **characterized in that** the first of the connection devices (200, 210) and the second of the connection devices (200, 210) are symmetric with respect to each other relative to the plane of symmetry (A).

9. The electrical component (2, 2A-2D) according to claim 7 or 8, **characterized in that** the outer shell (25) forms a planar abutment face (22) for placing the electrical component (2, 2A-2D) on another, second electrical component, wherein the plane of symmetry (A) is perpendicular to the abutment face (22).

10. The electrical component (2, 2A-2D) according to one of the preceding claims, **characterized in that** the first side face (20) and the second side face (21) each have a planar shape.

11. The electrical component (2, 2A-2D) according to one of the preceding claims, **characterized in that** the first side face (20) and the second side face (21) are arranged at an angle with respect to each other.

12. An implantable medical device (1), **characterized by** at least one electrical component (2, 2A-2D) according to one of the preceding claims.

13. The implantable medical device (1) according to claim 12, **characterized in that** the implantable medical device (1) comprises an assembly of a multiplicity of electrical components (2, 2A-2D) with outer shells (25) of equal shape, wherein the multiplicity of electrical components (2, 2A-2D) are assembled such that the outer shells (25) of the electrical components (2, 2A-2D) are in abutment with one another.

14. The implantable medical device (1) according to claim 13, **characterized in that** the first side face (20) of a first of the electrical components (2, 2A-2D) and the second side face (21) of a second of the electrical components (2, 2A-2D) are aligned to extend along a common plane.

15. The implantable medical device (1) according to one of claims 13 or 14, **characterized in that** said assembly forms the energy storage device (101) of the implantable medical device (1).

## Patentansprüche

1. Elektrische Komponente (2, 2A-2D) für ein implantierbares medizinisches Gerät (1), umfassend:
eine Außenschale (25), die eine erste Seitenfläche (20) und eine zweite Seitenfläche (21) ausbildet,
ein Funktionselement (26), das in der Außenschale (25) eingeschlossen ist, und eine Anordnung von Anschlussvorrichtungen (200, 210), angeordnet an der Außenschale (25), um eine elektrische Verbindung des Funktionselements (26) zu einer Komponente des implantierbaren medizinischen Geräts (1) außerhalb der Außenschale (25) herzustellen,
wobei eine erste der Anschlussvorrichtungen (200, 210) an der ersten Seitenfläche (20) und eine zweite der Anschlussvorrichtungen (200, 210) an der zweiten Seitenfläche (21) angeordnet ist, wobei die elektrische Komponente (2, 2A-2D) konfigurierbar ist, um verschiedene Konfigurationszustände anzunehmen, wobei in einem ersten Konfigurationszustand die erste der Anschlussvorrichtungen (200, 210) dafür konfiguriert ist, die genannte elektrische Verbindung herzustellen, und die zweite der Anschlussvorrichtungen (200, 210) deaktiviert ist, und in einem zweiten Konfigurationszustand die erste der Anschlussvorrichtungen (200, 210) deaktiviert ist und die zweite der Anschlussvorrichtungen (200, 210) dafür konfiguriert ist, die genannte elektrische Verbindung herzustellen, und **dadurch gekennzeichnet, dass**
die elektrische Komponente (2, 2A-2D) dafür verwendet wird, eine Energiespeichervorrichtung (101) für das implantierbare medizinische Gerät (1) zu bilden.

2. Elektrische Komponente (2, 2A-2D) nach Anspruch 1, **dadurch gekennzeichnet, dass** die Anschlussvorrichtungen (200, 210) durch Durchgangsöffnungen ausgebildet sind.

3. Elektrische Komponente (2, 2A-2D) nach Anspruch 1 oder 2, **durch** ein Schließelement (24) **gekennzeichnet,** um je nach Konfigurationszustand die erste oder die zweite der Anschlussvorrichtungen (200, 210) zu deaktivieren.

4. Elektrische Komponente (2, 2A-2D) nach einem der Ansprüche 1 bis 3, **dadurch gekennzeichnet, dass** zur Herstellung der genannten elektrischen Verbindung eine Anschlussleitung (102) durch die erste oder die zweite von den Anschlussvorrichtungen (200, 210), je nach Konfigurationszustand, verläuft.

5. Elektrische Komponente (2, 2A-2D) nach Anspruch 4, **durch** ein Führungselement (27) **gekennzeichnet,** um die Anschlussleitung (102) durch die erste oder die zweite von den Anschlussvorrichtungen (200, 210), je nach Konfigurationszustand, zu führen.

6. Elektrische Komponente (2, 2A-2D) nach Anspruch 4 oder 5, **dadurch gekennzeichnet, dass** das Funktionselement (26) an einem Anschlussort (260) innerhalb der Außenschale (25) mit der Anschlussleitung (102) verbunden ist, wobei der Anschlussort (260) in Bezug auf die erste Seitenfläche (20) und die zweite Seitenfläche (21) symmetrisch angeordnet ist.

7. Elektrische Komponente (2, 2A-2D) nach einem der vorstehenden Ansprüche, **dadurch gekennzeichnet, dass** die erste Seitenfläche (20) und die zweite Seitenfläche (21) so ausgebildet und angeordnet sind, dass die erste Seitenfläche (20) und die zweite Seitenfläche (21) zueinander relativ zu einer Symmetrieebene (A) symmetrisch sind.

8. Elektrische Komponente (2, 2A-2D) nach Anspruch 7, **dadurch gekennzeichnet, dass** die erste der Anschlussvorrichtungen (200, 210) und die zweite der Anschlussvorrichtungen (200, 210) zueinander relativ zu der Symmetrieebene (A) symmetrisch sind.

9. Elektrische Komponente (2, 2A-2D) nach Anspruch 7 oder 8, **dadurch gekennzeichnet, dass** die Außenschale (25) eine planare Anlagefläche (22) bildet, um die elektrische Komponente (2, 2A-2D) an einer weiteren, zweiten elektrischen Komponente zu platzieren, wobei die Symmetrieebene (A) senkrecht zur Anlagefläche (22) steht.

10. Elektrische Komponente (2, 2A-2D) nach einem der vorstehenden Ansprüche, **dadurch gekennzeichnet, dass** die erste Seitenfläche (20) und die zweite Seitenfläche (21) jeweils eine planare Form haben.

11. Elektrische Komponente (2, 2A-2D) nach einem der vorstehenden Ansprüche, **dadurch gekennzeichnet, dass** die erste Seitenfläche (20) und die zweite Seitenfläche (21) in einem Winkel zueinander angeordnet sind.

12. Implantierbares medizinisches Gerät (1), **durch** mindestens eine elektrische Komponente (2, 2A-2D) nach einem der vorstehenden Ansprüche **gekennzeichnet.**

13. Implantierbares medizinisches Gerät (1) nach Anspruch 12, **dadurch gekennzeichnet, dass** das implantierbare medizinisches Gerät (1) eine Baugruppe einer Vielzahl elektrischer Komponenten (2, 2A-2D) mit Außenschalen (25) gleicher Form umfasst, wobei die Vielzahl elektrischer Komponenten (2, 2A-2D) so zusammengebaut sind, dass die Außenschalen (25) der elektrischen Komponenten (2, 2A-2D) aneinander anliegen.

14. Implantierbares medizinisches Gerät (1) nach Anspruch 13, **dadurch gekennzeichnet, dass** die erste Seitenfläche (20) einer ersten der elektrischen Komponenten (2, 2A-2D) und die zweite Seitenfläche (21) einer zweiten der elektrischen Komponenten (2, 2A-2D) darauf ausgerichtet sind, entlang einer gemeinsamen Ebene zu verlaufen.

15. Implantierbares medizinisches Gerät (1) nach einem der Ansprüche 13 oder 14, **dadurch gekennzeichnet, dass** die genannte Baugruppe eine Energiespeichervorrichtung (101) des implantierbaren medizinischen Geräts (1) bildet.

## Revendications

1. Composant électrique (2, 2A-2D) pour un dispositif médical implantable (1), comprenant :
une coque externe (25) formant une première face latérale (20) et une seconde face latérale (21),
un élément fonctionnel (26) renfermé dans la coque externe (25), et
un agencement de dispositifs de connexion (200, 210) agencés sur la coque externe (25) pour établir une connexion électrique de l'élément fonctionnel (26) à un composant du dispositif médical implantable (1) à l'extérieur de la coque externe (25),
dans lequel un premier des dispositifs de connexion (200, 210) est agencé sur la première face latérale (20) et un second des dispositifs de connexion (200, 210) est agencé sur la seconde face latérale (21), dans lequel le composant électrique (2, 2A-2D) peut être configuré pour prendre différents états de configuration, dans lequel dans un premier état de configuration le premier des dispositifs de connexion (200, 210) est configuré pour établir ladite connexion électrique et le second des dispositifs de connexion (200, 210) est désactivé, et dans un second état de configuration, le premier des dispositifs de connexion (200, 210) est désactivé et le second des dispositifs de connexion (200, 210) est configuré pour établir ladite connexion électrique, et
**caractérisé en ce que**
le composant électrique (2, 2A-2D) est utilisé pour former un dispositif de stockage d'énergie (101) pour le dispositif médical implantable (1).

2. Composant électrique (2, 2A-2D) selon la revendication 1, **caractérisé en ce que** les dispositifs de connexion (200, 210) sont formés par des orifices traversants.

3. Composant électrique (2, 2A-2D) selon la revendication 1 ou 2, **caractérisé par** un élément de fermeture (24) pour désactiver, en fonction de l'état de configuration, le premier ou le second des dispositifs de connexion (200, 210).

4. Composant électrique (2, 2A-2D) selon l'une des revendications 1 à 3, **caractérisé en ce que**, pour établir ladite connexion électrique, une ligne de connexion (102) est passée à travers le premier ou le second des dispositifs de connexion (200, 210), en fonction de l'état de configuration.

5. Composant électrique (2, 2A-2D) selon la revendication 4, **caractérisé par** un élément de guidage (27) destiné à guider la ligne de connexion (102) à travers le premier ou le second des dispositifs de connexion (200, 210), en fonction de l'état de configuration.

6. Composant électrique (2, 2A-2D) selon la revendication 4 ou 5, **caractérisé en ce que** l'élément fonctionnel (26), au niveau d'un emplacement de connexion (260) à l'intérieur de la coque externe (25), est connecté à la ligne de connexion (102), dans lequel l'emplacement de connexion (260) est agencé symétriquement par rapport à la première face latérale (20) et à la seconde face latérale (21).

7. Composant électrique (2, 2A-2D) selon l'une des revendications précédentes, **caractérisé en ce que** la première face latérale (20) et la seconde face latérale (21) sont formées et agencées de telle sorte que la première face latérale (20) et la seconde face latérale (21) sont symétriques l'une de l'autre par rapport à un plan de symétrie (A).

8. Composant électrique (2, 2A-2D) selon la revendication 7, **caractérisé en ce que** le premier des dispositifs de connexion (200, 210) et le second des dispositifs de connexion (200, 210) sont symétriques l'un de l'autre par rapport au plan de symétrie (A).

9. Composant électrique (2, 2A-2D) selon la revendication 7 ou 8, **caractérisé en ce que** la coque externe (25) forme une face en butée plane (22) pour placer le composant électrique (2, 2A-2D) sur un autre second composant électrique, dans lequel le plan de symétrie (A) est perpendiculaire à la face en butée (22).

10. Composant électrique (2, 2A-2D) selon l'une des revendications précédentes, **caractérisé en ce que** la première face latérale (20) et la seconde face latérale (21) ont chacune une forme plane.

11. Composant électrique (2, 2A-2D) selon l'une des revendications précédentes, **caractérisé en ce que** la première face latérale (20) et la seconde face latérale (21) sont agencées à un angle l'un par rapport à l'autre.

12. Dispositif médical implantable (1), **caractérisé par** au moins un composant électrique (2, 2A-2D) selon l'une des revendications précédentes.

13. Dispositif médical implantable (1) selon la revendication 12, **caractérisé en ce que** le dispositif médical implantable (1) comprend un ensemble d'une multiplicité de composants électriques (2, 2A-2D) avec des coques externes (25) de forme égale, dans lequel la multiplicité de composants électriques (2, 2A-2D) sont assemblés de telle sorte que les coques externes (25) des composants électriques (2, 2A-2D) sont en butée l'une avec l'autre.

14. Dispositif médical implantable (1) selon la revendication 13, **caractérisé en ce que** la première face latérale (20) d'un premier des composants électriques (2, 2A-2D) et la seconde face latérale (21) d'un second des composants électriques (2, 2A-2D) sont alignées pour s'étendre le long d'un plan commun.

15. Dispositif médical implantable (1) selon l'une des revendications 13 ou 14, **caractérisé en ce que** ledit ensemble forme le dispositif de stockage d'énergie (101) du dispositif médical implantable (1).
